**Europäisches Patentamt**

**European Patent Office**

**Office européen des brevets**

(11) Veröffentlichungsnummer: **0 065 194**
**B1**

## (12) EUROPÄISCHE PATENTSCHRIFT

(45) Veröffentlichungstag der Patentschrift:
03.10.84

(51) Int. Cl.³: **C 07 C 11/12, C 07 C 7/08**

(21) Anmeldenummer: **82103839.5**

(22) Anmeldetag: **05.05.82**

(54) Verfahren zur Gewinnung eines konjugierten Diolefins aus einem C4- oder C5-Kohlenwasserstoffgemisch.

(30) Priorität: **16.05.81 DE 3119540**

(43) Veröffentlichungstag der Anmeldung:
**24.11.82 Patentblatt 82/47**

(45) Bekanntmachung des Hinweises auf die Patenterteilung:
**03.10.84 Patentblatt 84/40**

(84) Benannte Vertragsstaaten:
**AT BE DE FR GB IT NL SE**

(56) Entgegenhaltungen:
**DE - A - 1 443 987**
**DE - A - 1 807 675**
**US - A - 3 000 794**

**R. Billet "Industrielle Destillation" (1973) Seiten 376-378**

(73) Patentinhaber: **BASF Aktiengesellschaft,
Carl-Bosch-Strasse 38, D-6700 Ludwigshafen (DE)**

(72) Erfinder: **Broellos, Klaus, Dr., Floriansring 7,
D-6101 Seeheim (DE)**
Erfinder: **Volkamer, Klaus, Dr., Heidelberger Ring 21,
D-6710 Frankenthal (DE)**
Erfinder: **Lindner, Alfred, Dr., Ringstrasse 30,
D-6712 Bobenheim-Roxheim (DE)**

## Beschreibung

Die vorliegende Erfindung betrifft ein Verfahren zur Gewinnung eines konjugierten Diolefins aus einem dieses enthaltenden $C_4$- oder $C_5$-Kohlenwasserstoffgemisch durch extraktive Destillation mit Hilfe eines selektiven Lösungsmittels unter Regenerierung eines Teilstromes des im Kreise geführten selektiven Lösungsmittels.

Die extraktive Destillation ist ein bekanntes Verfahren zur Trennung von Gemischen, die durch übliche fraktionierte Destillation nicht leicht trennbar sind, z.B. wenn die zu trennenden Komponenten ein Azeotrop bilden oder geringe Unterschiede in den relativen Flüchtigkeiten besitzen. Bei der extraktiven Destillation wird in die Destillationskolonne eine solche Menge eines relativ schwer flüchtigen Lösungsmittels eingeführt, dass die Unterschiede in den relativen Flüchtigkeiten der zu trennenden Komponenten erhöht werden und damit eine destillative Trennung ermöglicht wird. Typische Anwendungsbeispiele für die extraktive Destillation finden sich beispielsweise in C.S. Robinson et al., Elements of Fractional Distillation", 4. Aufl. McGraw-Hill Book Company, Inc., New York (1959), S. 291.

Es ist bekannt, z.B. aus der DE-OS Nr. 2742148, DE-AS Nr. 1568902, DE-PS Nr. 1163795 oder aus „The Soviet Chemical Industry, Nr. 11, November 1971, S. 719 bis 723, konjugierte Diolefine aus einem $C_4$- oder $C_5$-Kohlenwasserstoffgemisch durch extraktive Destillation unter Verwendung eines selektiven Lösungsmittels zu gewinnen. Aufgrund der Neigung der in den $C_4$- oder $C_5$-Kohlenwasserstoffen enthaltenen höheren Acetylene und Allene wie Butenin, Butin, Butadien, Propin, Allen, sowie der konjugierten Diolefine zur Bildung von Polymeren bei der extraktiven Destillation können sich bei Rückführung des selektiven Lösungsmittels über einen längeren Zeitraum im selektiven Lösungsmittel Polymere ansammeln, die zu Polymerablagerungen in der Extraktionsanlage führen können („Oil & Gas Journal", Oktober 1979, S. 81 und 82). Es ist weiter bekannt, dem in der extraktiven Destillation verwendeten selektiven Lösungsmittel Polymerisationsinhibitoren zuzusetzen. Aber auch hierdurch kann die Polymerbildung nicht völlig vermieden werden („Oil & Gas Journal", Oktober 1979, S. 81 und 82).

Aus der US-PS Nr. 3000794, Spalte 5, ist bekannt, zur Vermeidung von Polymerisatablagerungen bei der Butadiengewinnung durch extraktive Destillation einen Teilstrom des im Kreise geführten selektiven Lösungsmittels kontinuierlich zu destillieren und das destillierte Lösungsmittel zum Hauptlösungsmittelstrom zurückzuführen. Durch die Abtrennung der im Teilstrom enthaltenen Polymerisate kann die Polymerisatkonzentration im selektiven Lösungsmittel in der Extraktionsanlage genügend niedrig gehalten werden, so dass Polymerisatablagerungen und somit Verstopfungen in der Extraktionsanlage vermieden werden können. Die Regenerierung des rückgeführten selektiven Lösungsmittels durch Destillation hat jedoch den Nachteil, dass hierbei erhebliche Verluste an wertvollem selektivem Lösungsmittel, das, um ein Ausfällen oder Auskristallisieren der abzutrennenden Verunreinigungen zu vermeiden, mit dem Sumpfprodukt der Destillation abgezogen wird, in Kauf genommen werden müssen. Diese Verluste sind besonders erheblich bei der Verwendung von Polymerisationsinhibitoren, die bei der Destillation zusätzlich zu den Polymerisaten aus dem zu regenerierenden Lösungsmittel abgetrennt werden und häufig besonders leicht zu Ausfällungen oder zum Auskristallisieren neigen.

Die vorliegende Erfindung soll nun eine Verbesserung der Arbeitsweise und Wirtschaftlichkeit der bekannten Verfahren bewirken.

Es wurde nun ein vorteilhaftes Verfahren gefunden zur Gewinnung eines konjugierten Diolefins aus einem dieses enthaltenden $C_4$- oder $C_5$-Kohlenwasserstoffgemisch durch ein oder mehrstufige extraktive Destillation mit einem selektiven Lösungsmittel und Rückführung des aus der extraktiven Destillation jeweils nachgeschalteten Ausstreifzone erhaltenen selektiven Lösungsmittels in die extraktive Destillation, wobei ein Teilstrom des rückgeführten selektiven Lösungsmittels in einer Lösungsmittelreinigungsstufe unter Abtrennung der im Lösungsmittel enthaltenen hochsiedenden oder nichtflüchtigen Verunreinigungen regeneriert wird und das aus der Lösungsmittelreinigungsstufe erhaltene regenerierte Lösungsmittel ebenfalls rückgeführt wird, welches dadurch gekennzeichnet ist, dass das zu regenerierende Lösungsmittel in einer Dünnschichtverdampfungszone regeneriert wird und dabei ein Ausstreifmittel verwendet wird.

Vorzugsweise wird bei dem erfindungsgemässen Verfahren das zu regenerierende Lösungsmittel im oberen Drittel, zweckmässig am Kopf, einer senkrecht angeordneten Dünnschichtverdampfungszone zugeführt, aus der das regenerierte Lösungsmittel als Kopfprodukt und ein die hochsiedenden und nichtflüchtigen Verunreinigungen enthaltendes Sumpfprodukt abgezogen werden.

Nach dem erfindungsgemässen Verfahren lassen sich die Verluste an selektivem Lösungsmittel wesentlich verringern. Das Verfahren ist besonders geeignet für die Regenerierung von Polymerisationsinhibitoren enthaltenden selektiven Lösungsmitteln, da diese Systeme, z.B. bei der Verwendung von Nitrogruppen enthaltenden Polymerisationsinhibitoren, bei der Aufkonzentrierung aufgrund einer möglichen Labilität besondere Vorsichtsmassnahmen gegen spontane Zersetzung erforderlich machen können.

Die erfindungsgemässe Regenerierung erfolgt in einer Dünnschichtverdampfungszone, die zweckmässig senkrecht angeordnet ist. Hierzu verwendet man im allgemeinen senkrecht angeordnete Dünnschichtverdampfer, die zweckmässig als senkrecht angeordnete Kolonnen ausgestaltet sind, deren innere Oberfläche beheizbar ist, z.B. durch eine Elektroheizung oder durch indirekte Beheizung mit einer Heizflüssigkeit, wie heisses Wasser oder Diphenyl, oder vorzugsweise mit Dampf. Es kann vorteilhaft sein, zur besseren Verteilung des zu regenerierenden selektiven Lösungsmittels auf der beheizten inneren Oberfläche des Dünnschichtverdampfers und um die Bildung von eventuellen Ablagerungen auf der Heizfläche zu vermeiden, mechanisch bewegte Wischer zu verwenden, die die Heizfläche des Dünnschichtverdampfers teilweise oder vorzugsweise ganz bestreichen. Dabei können die Wischer an der Wand entlang streifen oder in einem geringen Abstand von der Wand, z.B. im Abstand von 0,5 bis 10, vorzugsweise 0,6 bis 5, insbesondere 0,5 bis 2 mm, geführt werden. Die Wischer können beispielsweise an einer im Dünnschichtverdampfer senkrecht angeordneten Welle befestigt sein und werden zweckmässig durch Rotation der Welle bewegt. Geeignete Dünnschichtverdampfer sind beispielsweise als Sambay- oder Luwa-Dünnschichtverdampfer bekannt.

Geeignete selektive Lösungsmittel für das erfindungsgemässe Verfahren sind beispielsweise Butyrolacton, Furfurol, Methoxyproprionitril und vorzugsweise N-alkylsubstituierte niedere aliphatische Säureamide wie Dimethylformamid, Diethylformamid, Dimethylacetamid, Diethylacetamid oder N-Formylmorpholin und N-alkylsubstituierte cyclische aliphatische Säureamide (Lactame) mit 5 Ringgliedern wie N-Alkylpyrrolidone mit 1 bis 3 Kohlenstoffatomen im Alkylrest, insbesondere N-Methylpyrrolidon. Mit besonderem Vorteil werden von den selektiven Lösungsmitteln Dimethylformamid und insbesondere N-Methylpyrrolidon verwendet.

Im allgemeinen beträgt der Teilstrom des rückgeführten selektiven Lösungsmittel, der in der Lösungsmittelreinigungsstufe regeneriert wird, 0,01 bis 5, vorzugsweise 0,01 bis 2, insbesondere 0,04 bis 1 Gew.-% der Menge des in die extraktive Destillation rückgeführten selektiven Lösungsmittels.

Bei den aus dem selektiven Lösungsmittel in der Regenerierstufe abzutrennenden hochsiedenden oder nichtflüchtigen Verunreinigungen handelt es sich im allgemeinen um in der Extraktionsanlage, z.B. durch Sauerstoffeintritt in die Extraktionsanlage, gebildete Polymerisate, um dem selektiven Lösungsmittel zugefügte Polymerisationsinhibitoren und bzw. oder gegebenenfalls dem selektiven Lösungsmittel zugefügte Antischaummittel, z.B. Silikonöl. Die hochsiedenden Verunreinigungen haben im allgemeinen einen Siedepunkt von mindestens 300, in der Regel von mindestens 400°C.

Geeignete Polymerisationsinhibitoren, die dem selektiven Lösungsmittel zugesetzt werden können, sind z.B. Furfurol, Benzaldehyd, $\beta$-ungesättigte Nitrile, aromatische Merkaptane, aliphatische Nitroverbindungen, Zimtaldehyd, Aldol, Nitroso-$\beta$-naphthol, Isatin, Morpholin, aliphatische tert.-Merkaptane, Alkylnitrit, Methylenblau, Natriumnitrit, Hydrochinon, Schwefel, phenolische Verbindungen, wie 4-tert.-Butylcatechol, aromatische Amine, wie $\beta$-Naphthylamin, aromatische Nitroverbindungen, wie Nitrobenzol, Nitronaphthalin oder kernsubstituierte Derivate davon (DE-PS Nr. 1816826).

Erfindungsgemäss wird die Regenerierung des selektiven Lösungsmittels unter Verwendung eines Ausstreifmittels für das Ausstreifen des selektiven Lösungsmittels durchgeführt. Vorteilhaft werden solche Ausstreifmittel für die Regenerierung in der Dünnschichtverdampfungszone verwendet, welche als Zusatz zum selektiven Lösungsmittel die Wirksamkeit des selektiven Lösungsmittels bei der extraktiven Destillation verbessern, indem sie z.B. die $C_4$- oder $C_5$-Kohlenwasserstoffselektivität des selektiven Lösungsmittels erhöhen und/oder die Viskosität des selektiven Lösungsmittels verringern und/oder den Siedepunkt des selektiven Lösungsmittels herabsetzen. Vorzugsweise wird als Ausstreifmittel Wasser oder ein organisches Lösungsmittel mit einem Siedepunkt im Bereich von 30 bis 200, vorzugsweise 40 bis 150, insbesondere 45 bis 125°C, verwendet. Das Ausstreifmittel kann dem zu regenerierenden selektiven Lösungsmittel, das der Dünnschichtverdampfungszone zugeführt wird, zugefügt sein. Vorzugsweise wird das Ausstreifmittel im unteren Drittel der Dünnschichtverdampfungszone zugeführt, wobei zusätzlich zu dem im unteren Drittel zugeführten Ausstreifmittel noch Ausstreifmittel im zu regenerierenden selektiven Lösungsmittel, das der Dünnschichtverdampfungszone zugeführt wird, enthalten sein kann. Vorzugsweise wird das im unteren Drittel der Dünnschichtverdampfungszone zugeführte Ausstreifmittel im Sumpf der Dünnschichtverdampfungszone zugeführt. Im allgemeinen beträgt das Verhältnis der Menge an insgesamt im zu regenerierenden Lösungsmittel und/oder im unteren Drittel der Dünnschichtverdampfungszone zugeführtem Ausstreifmittel zu der Menge des zu regenerierenden selektiven Lösungsmittels 1:100 bis 10:1, vorzugsweise 1:50 bis 5:1, insbesondere 1:20 bis 1:1.

Für die Regenerierung des selektiven Lösungsmittels in der Dünnschichtverdampfungszone werden im allgemeinen Temperaturen zwischen 0 und 220°C angewendet. Mit besonderem Vorteil werden in der Dünnschichtverdampfungszone zwei Temperaturzonen mit unterschiedlichen Temperaturen aufrechterhalten, und zwar in der Weise, dass im Sumpf der Dünnschichtverdampfungszone niedrigere Temperaturen als in der Heizzone der Dünnschichtverdampfungszone eingehalten werden. Im allgemeinen werden im Sumpf der Dünnschichtverdampfungszone Temperaturen zwischen 0 und 120, vorzugsweise 0 und 100, insbesondere 0 und 60°C, aufrechterhalten, während in der Heizzone des Dünnschichtverdampfers, d.h. der Zone des Dünnschichtverdampfers, in der die innere Oberfläche beheizt, wird zweckmässig Temperaturen zwischen 30 und 220, vorzugsweise zwischen 50 und 200, insbesondere zwischen 70 und 180°C angewendet werden. Man kann die Regenerierung bei Atmosphärendruck oder leicht erhöhtem Druck durchführen. Vorzugsweise wendet man verminderten Druck, im allgemeinen Drücke zwischen 1 und 990, vorzugsweise zwischen 5 und 500, insbesondere zwischen 10 und 200 mbar an.

Das aus der Dünnschichtverdampfungszone abgezogene regenerierte selektive Lösungsmittel wird in die extraktive Destillation zurückgeführt.

Zweckmässig wird es hierzu dem Hauptstrom des rückgeführten selektiven Lösungsmittels zugeführt. Falls das Ausstreifmittel gleichzeitig als Zusatz zum selektiven Lösungsmittel verwendet wird, wird man das Ausstreifmittel vorzugsweise in solcher Menge verwenden, dass die Menge des Ausstreifmittels im aus der Dünnschichtverdampfungszone abgezogenen regenerierten selektiven Dünnschichtverdampfungszone prozentual etwa von der gleichen Grössenordnung ist, wie die Menge des Zusatzes in dem in der extraktiven Destillation verwendeten selektiven Lösungsmittels. Im allgemeinen beträgt der Prozentgehalt an Ausstreifmittel im aus der Dünnschichtverdampfungszone abgezogenen regenerierten selektiven Lösungsmittel höchstens das 5fache, vorzugsweise höchstens das 2fache und mindestens das 0,1fache, vorzugsweise mindestens das 0,5fache des Prozentgehaltes des Zusatzes in dem in der extraktiven Destillation verwendeten selektiven Lösungsmittel.

Am Sumpf der Dünnschichtverdampfungszone wird zweckmässig ein die hochsiedenden oder nichtflüchtigen Verunreinigungen enthaltendes Sumpfprodukt abgezogen, welches höchstens 15, vorzugsweise höchstens 10, insbesondere höchstens 5 Gew.-% an selektivem Lösungsmittel, bezogen auf das abgezogene Sumpfprodukt, enthält. Im allgemeinen dient das verwendete Ausstreifmittel gleichzeitig als Verdünnungsmittel für das abgezogene Sumpfprodukt. Zweckmässig wird ein Sumpfprodukt abgezogen, welches 5 bis 99, vorzugsweise 10 bis 98, insbesondere 20 bis 98 Gew.-%, an Ausstreifmittel, bezogen auf das abgezogene Sumpfprodukt enthält.

Es kann vorteilhaft sein, einen Teil des aus dem Sumpf der Dünnschichtverdampfungszone abgezogenen Sumpfproduktes im Kreis zum Sumpf der Dünnschichtverdampfungszone zurückzuführen. Im allgemeinen wird ein Teilstrom zum Sumpf zurückgeführt, der 1 bis 99, vorzugsweise 5 bis 95, insbesondere 10 bis 90 Gew.-% der Menge des aus dem Sumpf der Dünnschichtverdampfungszone abgezogenen Sumpfproduktes beträgt. Durch diese Arbeitsweise kann die Ablagerung von Niederschlägen im Sumpf der Dünnschichtverdampfungszone vermieden werden.

Die Lösungsmittel, die beim erfindungsgemässen Verfahren als Ausstreifmittel in der Dünnschichtverdampfungszone und/oder als Zusatz zum selektiven Lösungsmittel in der extraktiven Destillation verwendet werden können, sind Wasser und/oder ein organisches Lösungsmittel mit einem Siedepunkt im Bereich von 30 bis 200, vorzugsweise 50 bis 160°C.

Geeignete als Ausstreifmittel und/oder Zusatz zum selektiven Lösungsmittel zu verwendende organische Lösungsmittel sind beispielsweise Nitrile, aliphatische oder alicyclische Ether, niedere aliphatische Carbonsäureester oder Kohlensäureester, Amine, Alkohole, aliphatische Ketone, Etheramine, aliphatische Säureamide mit einem Siedepunkt im Bereich von 30 bis 200°C. Als Nitrile kommen beispielsweise Butyronitril, Propionitril und vorzugsweise Acetonitril in Betracht. Als aliphatische Ether kommen beispielsweise symmetrische oder unsymmetrische Ether der allgemeinen Formel R−O−R' in Betracht, in denen im allgemeinen der aliphatische Rest R ein Kohlenwasserstoffrest mit 1 bis 6, vorzugsweise 1 bis 5 Kohlenstoffatomen und der aliphatische Rest R' ein Kohlenwasserstoffrest mit 2 bis 6, vorzugsweise 2 bis 5 Kohlenstoffatomen ist. Die Gesamtzahl der Kohlenstoffatome beider Kohlenwasserstoffreste beträgt zweckmässig 4 bis 12, vorzugsweise 5 bis 10. Geeignete Reste R und R' sind z.B. der Ethyl-, n-Propyl-, Isopropyl-, n-Butyl-, Isobutyl-, tert.-Butylrest, die Amylreste wie tert.-Amylrest, der Hexylrest, wobei R zusätzlich einen Methylrest bedeuten kann. Geeignete Ether sind beispielsweise symmetrische Ether wie Diethylether, Di-n-propylether, Diisopropylether, Di-n-butylether, Diisobutylether, sowie vorzugsweise unsymmetrische Ether wie Methyl-n-butylether, Methyl-tert.-butylether, Ethyl-tert.-butylether, n-Propyl-tert.-butylether, Isopropyl-tert.-butylether, Isobutyl-tert.-butylether, n-Butyl-tert.-butylether, Methyl-tert.-amylether, Ethyl-tert.-amylether, N-Propyl-tert.-amylether, Ethylisopropylether, n-Propyliso-propylether, n-Butylisopropylether, Isobutyliso-propylether, Ethyl-n-butylether. Besonders vorteilhaft werden von den unsymmetrischen Ethern solche verwendet, in denen ein Rest einer tert.-Amyl- oder insbesondere einer tert.-Butylgruppe ist. Weitere geeignete aliphatische Ether sind z.B. Ethylenglykolmonomethylether sowie vorzugsweise die Dialkylether von Ethylenglykol oder 1,2-Propylenglykol wie Ethylenglykoldimethylether, -diethylether, -methylethylether, -methylisopropylether, 1,2-Propylenglykoldimethylether. Als alicyclische Ether kommen beispielsweise 2-Methyltetrahydrofuran, 3-Methyltetrahydrofuran, 1,4-Dioxan, Tetrahydropyran und vorzugsweise Tetrahydrofuran in Betracht. Als niedere aliphatische Carbonsäureester kommen beispielsweise solche in Betracht, die sich von niederen Monocarbonsäuren mit im allgemeinen 1 bis 4, vorzugsweise 1 bis 3 Kohlenstoffatomen ableiten wie Ameisensäure, Essigsäure, Propionsäure oder von Dicarbonsäuren mit 2 oder 3 Kohlenstoffatomen, wie Oxalsäure oder Malonsäure. Die Alkoholkomponente leitet sich im allgemeinen ab von einwertigen Alkoholen mit zweckmässig 1 bis 6, vorzugsweise 1 bis 4 Kohlenstoffatomen wie Methanol, Ethanol, Propanol, Isopropanol, n-Butanol, Isobutanol oder von zweiwertigen Alkoholen mit im allgemeinen 2 oder 3 Kohlenstoffatomen, wie Ethylenglykol, 1,2-Propylenglykol. Geeignete niedere aliphatische Carbonsäureester sind z.B. Ameisensäureester wie Ameisensäuremethylester, Ameisensäureethylester, Ameisensäurepropyles-ter, Ameisensäureisopropylester, Ameisensäure-butylester, Ameisensäureisobutylester, Ameisen-säureamylester; Essigsäureester wie Essigsäure-methylester, Essigsäureethylester, Essigsäure-butylester, Ethylenglykoldiacetat; Propionsäure-ethylester, Propionsäureamylester; Malonsäure-dimethylester, Oxalsäurediethylester. Als Kohlen-säureester kommen beispielsweise der Kohlen-säuremethylethylester, Kohlensäuredimethylester,

Kohlensäuredibutylester in Betracht. Als Amine kommen beispielsweise in Betracht primäre aliphatische Amine mit aliphatischen Kohlenwasserstoffresten mit 4 bis 5 Kohlenstoffatomen wie n-Butylamin, sek.-Butylamin, Isobutylamin, n-Amylamin, Isoamylamin, tert.-Amylamin, sek.-n-Amylamin; sekundäre aliphatische Amine mit aliphatischen Kohlenwasserstoffresten mit insgesamt 4 bis 6 Kohlenstoffatomen wie Diethylamin, Dipropylamin, Diisopropylamin, Piperidin; tertiäre aliphatische Amine mit aliphatischen Kohlenwasserstoffresten mit insgesamt 5 bis 6 Kohlenstoffatomen wie N,N-Dimethylisobutylamin, Triethylamin. Geeignete Alkohole sind z.B. solche mit 1 bis 5 Kohlenstoffatomen wie Methanol, Ethanol, Propanol, Isopropanol, Butanol, Isobutanol oder tert.-Amylalkohol, wobei Isopropanol bevorzugt wird. Als Ketone kommen beispielsweise aliphatische Ketone mit insgesamt 3 bis 5 Kohlenstoffatomen wie Aceton, Methylethylketon, Diethylketon, Methylpropylketon in Betracht. Geeignete Etheramine sind z.B. Alkoxyalkylamin mit insgesamt 3 bis 4 Kohlenstoffatomen wie 2-Methoxyethylamin, 2-Ethoxyethylamin, 3-Methoxypropylamin. Als aliphatische Säureamide kommen beispielsweise Dimethylformamid, Diethylformamid in Betracht. Mit besonderem Vorteil werden von den organischen Lösungsmitteln die Ester, Dimethylformamid, vorzugsweise die Nitrile, insbesondere die aliphatischen oder alicyclischen Ether, verwendet.

Falls das organische Lösungsmittel als Zusatz zum selektiven Lösungsmittel verwendet wird, enthält die Lösungsmittelmischung aus Zusatz Lösungsmittel und selektivem Lösungsmittel im allgemeinen 1 bis 50, vorzugsweise 2 bis 25, insbesondere 2 bis 20 Gew.%, an Zusatzlösungsmittel. Entsprechend weist die Lösungsmittelmischung im allgemeinen einen Gehalt von 50 bis 99, vorzugsweise 75 bis 98, insbesondere 80 bis 97 Gew.-%, an selektivem Lösungsmittel auf. Mit besonderem Vorteil wird eine Lösungsmittelmischung gemäss der Erfindung verwendet, die 5 bis 15 Gew.-% an Zusatzlösungsmittel enthält. Die Lösungsmittelmischung kann einen geringen Wassergehalt, z.B. bis zu 10 Gew.-%, aufweisen. Zweckmässig wird jedoch der Wassergehalt auf höchstens 5, vorzugsweise höchstens 3 Gew.-%, bezogen auf die Lösungsmittelmischung begrenzt. Es kann jedoch auch vorteilhaft sein, im wesentlichen wasserfrei, d.h. mit einem Wassergehalt von höchstens 1, vorzugsweise höchstens 0,5, insbesondere höchstens 0,1 Gew.-%, bezogen auf die Lösungsmittelmischung, zu arbeiten. Bei Verwendung eines Zusatzlösungsmittels, welches, wie z.B. Acetonitril, mit Wasser ein azeotropes Gemisch bildet, kann es weiter vorteilhaft sein, eine Lösungsmittelmischung mit einem Wassergehalt anzuwenden, der etwa dem azeotropen Verhältnis von Wasser zu dem organischen Lösungsmittel, bezogen auf das in der Lösungsmittelmischung enthaltene organische Lösungsmittel, entspricht. Bei Wasserzugabe zur erfindungsgemässen Lösungsmittelmischung und Verwendung von Acetonitril als Zusatzlösungsmittel beträgt das Gewichtsverhältnis von Wasser zu Acetonitril in der Lösungsmittelmischung zweckmässig 1:3 bis 1:9, vorzugsweise 1:4 bis 1:8.

Die Gewinnung eines konjugierten Diolefins aus einem dieses enthaltenden $C_4$- oder $C_5$-Kohlenwasserstoffgemisch unter Verwendung der erfindungsgemäss als selektives Lösungsmittel einzusetzenden Lösungsmittelmischung erfolgt in an sich bekannter Weise (vgl. z.B. DE-PS Nr. 1184334, DE-AS Nr. 1568876, DE-AS Nr. 1568902) durch ein- oder mehrstufige, zweckmässig ein- oder zweistufige extraktive Destillation. Die Gewinnung der konjugierten Diolefine, wie 1,3-Butadien, Isopren und 1,3-Pentadien aus der $C_4$- oder $C_5$-Kohlenwasserstoffmischung erfolgt beispielsweise in der Weise, dass das $C_4$- oder $C_5$-Kohlenwasserstoffgemisch, welches löslichere Kohlenwasserstoffe als das konjugierte Diolefin und weniger lösliche Kohlenwasserstoffe als das konjugierte Diolefin enthält, einer extraktiven Destillation mit dem zu verwendenden Lösungsmittelgemisch unterworfen wird, wobei ein die weniger löslichen Kohlenwasserstoffe enthaltendes Destillat und ein das konjugierte Diolefin und die löslichen Kohlenwasserstoffe und das selektive Lösungsmittel enthaltender Extrakt abgetrennt werden. Aus dem Extrakt kann das konjugierte Diolefin als Rohprodukt isoliert werden, das für manche Verwendungszwecke eine ausreichende Reinheit aufweist, das jedoch auch noch weiteren Reinigungsoperationen, z.B. einer fraktionierten Destillation, unterworfen werden kann. Vorteilhaft wird das konjugierte Diolefin jedoch unter Anwendung von zwei hintereinandergeschalteten extraktiven Destillationsstufen unter Verwendung der Lösungsmittelmischung gewonnen.

Hierbei werden beispielsweise — wie bereits vorstehend beschrieben — in der ersten Stufe der extraktiven Destillation ein die weniger löslichen Kohlenwasserstoffe enthaltendes Destillat und ein das konjugierte Diolefin und die löslicheren Kohlenwasserstoffe und das selektive Lösungsmittel enthaltender Extrakt abgetrennt. Dieser Extrakt wird in einer Ausstreifzone vom selektiven Lösungsmittel befreit, wobei ein Gemisch aus dem konjugierten Diolefin und den löslicheren Kohlenwasserstoffen erhalten wird. Dieses Gemisch wird einer zweiten extraktiven Destillation mit dem selektiven Lösungsmittel unterworfen, wobei das konjugierte Diolefin als Destillat und ein Extrakt erhalten werden, der die löslicheren Kohlenwasserstoffe und das elektive Lösungsmittel enthält. Der erhaltene Extrakt wird anschliessend in einer Ausstreifzone vom selektiven Lösungsmittel befreit, wobei ein die löslicheren Kohlenwasserstoffe enthaltender Kohlenwasserstoffstrom erhalten wird.

Als konjugierte Diolefine enthaltendes Kohlenwasserstoffgemisch, das als Ausgangsgemisch für das Verfahren der vorliegenden Erfindung geeignet ist, kann eine $C_4$- oder $C_5$-Fraktion, die durch thermisches Kracken einer Petroleumfraktion (z.B. LPG, Naphtha usw.) erhalten wurde, eine durch Dehydrierung von n-Butan und/oder n-Buten erhaltene Butadien enthaltende Fraktion und eine durch Dehydrierung von Isopentan und/oder Isoamylen erhaltene Isopren enthaltende Fraktion verwendet werden. Im allgemeinen enthält das $C_4$-Kohlenwasserstoffgemisch 1,3-Butadien als konjugiertes Diolefin, Butane, n-Buten, Isobuten, Vinylacetylen, Ethylacetylen, 1,2-Butadien und gegebenenfalls geringe Mengen $C_3$- und/oder $C_5$-Kohlenwasserstoffe. Das $C_5$-Kohlenwasserstoffgemisch enthält in der Regel Isopren, trans- und cis-1,3-Pentadien und Cyclopentadien als konjugierte Diolefine sowie Pentane, n-Pentene, Isoamylen, Cyclopenten, höhere Acetylene.

Beispielsweise ergibt die extraktive Destillation einer $C_4$-Fraktion zunächst ein die Butane und Butene enthaltendes Destillat sowie einen 1,3-Butadien, Ethylenacetylen, Vinylacetylen und 1,2-Butadien enthaltenden Extrakt, der, wenn er einer weiteren extraktiven Destillation unterworfen wird, als Destillat 1,3-Butadien liefert, während der Extrakt Ethylacetylen, Vinylacetylen und 1,2-Butadien enthält. Aus dem Ethylacetylen, Vinylacetylen und 1,2-Butadien enthaltenden Extrakt werden diese Kohlenwasserstoffe in einem Ausgaser abgetrennt und das entgaste Lösungsmittel in die extraktive Destillation zurückgeführt. Das als Destillat erhaltene 1,3-Butadien kann anschliessend zur Abtrennung von gegebenenfalls noch vorhandenen sehr geringen Mengen von $C_3$- und/oder $C_5$-Kohlenwasserstoffen einer fraktionierten Destillation unterworfen werden.

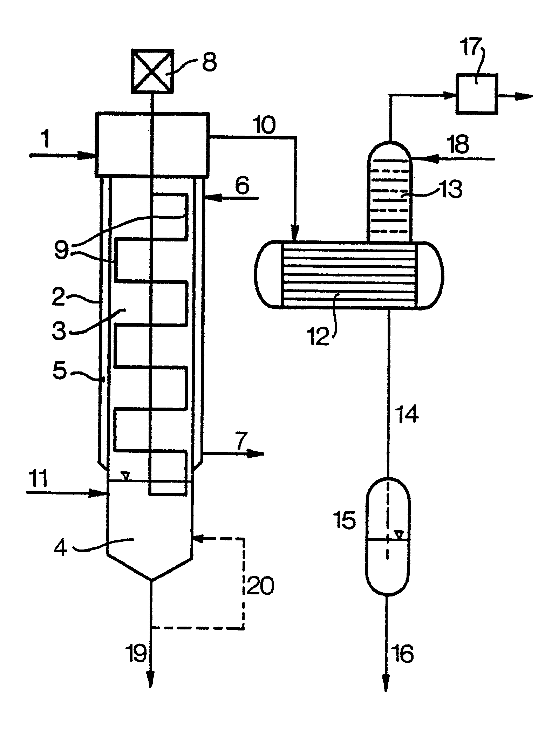

Die Figur ist ein schematisches Diagramm einer Ausführungsform des erfindungsgemässen Verfahrens. Das zu regenerierende selektive Lösungsmittel, z.B. eine Mischung aus N-Methylpyrrolidon und 5 bis 15 Gew.-% Wasser, das aus einer Anlage zur Gewinnung von Butadien aus einem $C_4$-Kohlenwasserstoffgemisch erhalten wird, wird über Leitung 1 einem kolonenförmigen Dünnschichtverdampfer 2 zugeführt und durch die durch Motor 8 bewegten Wischer 9 auf der inneren beheizten Fläche der Heizzone 3 des Dünnschichtverdampfers verteilt und verdampft. Die Beheizung der Heizfläche erfolgt indirekt durch Einleiten von Dampf über Leitung 6 in den Heizmantel 5, aus dem über Leitung 7 flüssiges Kondensat abgezogen wird. Über Leitung 11 wird dem Sumpf 4 des Dünnschichtverdampfers ein Ausstreifmittel, z.B. Wasser, zugeführt, welches aus dem Sumpfprodukt noch vorhandene Reste an selektivem Lösungsmittel ausstreift und gleichzeitig als Verdünnungsmittel für das Sumpfprodukt dient. In der Heizzone 3 wird eine höhere Temperatur als im Sumpf 4 aufrechterhalten, z.B. eine Temperatur zwischen 120 und 150°C in der Heizzone und eine Temperatur zwischen beispielsweise 10 und 50°C im Sumpf des Dünnschichtverdampfers. Der Dünnschichtverdampfer wird unter vermindertem Druck, z.B. einem Druck von 10 bis 80 mbar, betrieben. Am Kopf des Dünnschichtverdampfers werden das verdampfte selektive Lösungsmittel und gegebenenfalls der verdampfte Zusatz zum selektiven Lösungsmittel, z.B. Wasser, sowie das verdampfte Ausstreifmittel, z.B. Wasser, über Leitung 10 abgezogen und im Kühler 12 kondensiert. Das Kondensat, frei von hochsiedenden oder flüchtigen Verunreinigungen, wird aus dem Kühler 12 über Leitung 14 in den Behälter 15 und von dort über Leitung 16 in die Butadiengewinnungsanlage zurückgeführt. Spuren des selektiven Lösungsmittels und anderer flüchtiger Komponenten werden auf dem Weg zum Vakuumsystem 17 in der Kolonne 13, der über Leitung 18 ein Waschmittel, z.B. Wasser, zugeführt wird, zurückgehalten. Der Sumpf des Dünnschichtverdampfers nimmt alle Verunreinigungen in noch flüssiger, pumpfähiger Phase auf. Der Flüssigkeitsstand im Sumpf wird, zweckmässig standgeregelt, im allgemeinen am unteren Rand der Heizzone 3 anschliessend gehalten, wodurch ein Auskristallisieren oder eine Fällung der Verunreinigungen verhindert werden kann. Das Sumpfprodukt, aus dem das selektive Lösungsmittel durch über Leitung 11 zugeführtes Wasser oder organisches Lösungsmittel praktisch vollständig ausgestreift und das gleichzeitig durch das zugeführte Wasser oder organische Lösungsmittel verdünnt wird, wird in flüssiger Form über Leitung 19 abgezogen. Es kann vorteilhaft sein, einen Teilstrom des abgezogenen Sumpfproduktes über Leitung 20 zum Sumpf 4 zurückzuführen.

Die nachstehenden Beispiele dienen der weiteren Erläuterung der Erfindung.

*Beispiel 1*

In einem kontinuierlich betriebenen, mit einem Rührwerk ausgestatten Dünnschichtverdampfer wurden 1200 g/h N-Methylpyrrolidon (NMP) mit einem Gehalt von 9,5 Gew.-% Wasser und 0,2 Gew.-% nichtflüchtigen Verunreinigungen, das aus dem rückgeführten Lösungsmittelstrom einer Butadienanlage entnommen wurde, bei einer Temperatur von 140°C und unter vermindertem Druck von 40 mbar und einer Rührerdrehzahl von 200 tr/min regeneriert. In den Sumpf des Dünnschichtverdampfers, der bei einer Temperatur von 30°C gehalten wurde, wurden 60 g/h Wasser zugegeben, das gleichzeitig als Ausstreifmittel für das NMP und als Verdünnungsmittel für das Sumpfprodukt diente. Als Destillat wurde reines NMP mit einem Wassergehalt von 10,1 Gew.-% erhalten. Das abgezogene Sumpfprodukt enthielt die gesamten nichtflüchtigen Verunreinigungen und eine Restgehalt an NMP von 0,53 Gew.-%, d.h. das selektive Lösungsmittel wurde zu 99,9% zurückgewonnen.

*Beispiele 2 und 3*

Man verfährt wie in Beispiel 1 beschrieben, wobei unter Verwendung der in Tabelle 1 angegebenen Ausgangsgemische die in Tabelle genannten Ergebnisse erhalten werden.

*(Tabelle auf der nächsten Seite)*

*Beispiel 4*

In einem kontinuierlich betriebenen Dünnschichtverdampfer wurden den Bedingungen von Beispiel 1 entsprechend 320 kg/h N-Methylpyrrolidon (NMP) mit einem Gehalt von 9,5 Gew.-

*Tabelle*

| Beispiel | Ausgangsgemisch NMP mit 9,5 Gew.-% $H_2O$ | | Destillat | | Wasser-zugabe (g/h) | NMP im Sumpf-produkt (Gew.-%) | NMP-Rück-gewinnungs-rate (%) |
| | (g/h) | Gehalt an nicht-flüchtigen Ver-unreinigungen | NMP (Gew.-%) | $H_2O$ (Gew.-%) | | | |
|---|---|---|---|---|---|---|---|
| 2 | 1104 | 1,7 Gew.-% | 87,7 | 12,3 | 4 | 0,41 | 98,8 |
| 3 | 863 | 0,2 Gew.-% | 89,0 | 11,0 | 8 | 0,06 | 98,7 |

% Wasser und 0,2 Gew.-% nichtflüchtigen Verunreinigungen, das aus einer Butadienextraktionsanlage erhalten wurde, unter Zugabe von 12,3 kg/h Wasser zum Sumpf des Dünnschichtverdampfers regeneriert. Dabei wurden 11,14 kg/h Sumpfprodukt mit einem Gehalt von 0,055 kg/h NMP und 0,64 kg/h nichtflüchtigen Verunreinigungen erhalten. Das NMP wurde zu 99,9% zurückgewonnen.

**Patentansprüche**

1. Verfahren zur Gewinnung eines konjugierten Diolefins aus einem dieses enthaltenden $C_4$- oder $C_5$-Kohlenwasserstoffgemisch durch ein- oder mehrstufige extraktive Destillation mit einem selektiven Lösungsmittel und Rückführung des aus der der extraktiven Destillation jeweils nachgeschalteten Ausstreifzone erhaltenen selektiven Lösungsmittels in die extraktive Destillation, wobei ein Teilstrom des rückgeführten selektiven Lösungsmittels in einer Lösungsmittelreinigungsstufe unter Abtrennung der im Lösungsmittel enthaltenen hochsiedenden oder nichtflüchtigen Verunreinigungen regeneriert wird und das aus der Lösungsmittelreinigungsstufe erhaltene regenerierte Lösungsmittel ebenfalls rückgeführt wird, dadurch gekennzeichnet, dass das zu regenerierende Lösungsmittel in einer Dünnschichtverdampfungszone regeneriert wird und dabei ein Ausstreifmittel verwendet wird.

2. Verfahren nach Anspruch 1, dadurch gekennzeichnet, dass das zu regenerierende selektive Lösungsmittel im oberen Drittel einer senkrecht angeordneten Dünnschichtverdampfungszone zugeführt wird, aus der das regenerierte Lösungsmittel als Kopfprodukt und ein die hochsiedenden oder nichtflüchtigen Verunreinigungen enthaltendes Sumpfprodukt abgezogen werden.

3. Verfahren nach den Ansprüchen 1 und 2, dadurch gekennzeichnet, dass das Ausstreifmittel im unteren Drittel der Dünnschichtverdampfungszone zugeführt wird.

4. Verfahren nach den Ansprüchen 1 bis 3, dadurch gekennzeichnet, dass das Ausstreifmittel im Sumpf der Dünnschichtverdampfungszone zugeführt wird.

5. Verfahren nach den Ansprüchen 1 bis 4, dadurch gekennzeichnet, dass als Ausstreifmittel Wasser oder organische Lösungsmittel mit einem Siedepunkt im Bereich von 30 bis 200°C verwendet werden.

6. Verfahren nach den Ansprüchen 1 bis 5, dadurch gekennzeichnet, dass ein Teil des aus dem Sumpf der Dünnschichtverdampfungszone abgezogenen Sumpfproduktes im Kreis zum Sumpf der Dünnschichtverdampfungszone zurückgeführt wird.

7. Verfahren nach den Ansprüchen 1 bis 6, dadurch gekennzeichnet, dass die Regenerierung in einem senkrecht angeordneten Dünnschichtverdampfer und unter Verwendung von mechanisch bewegten Wischern, welche die innere Wand des Dünnschichtverdampfers bestreichen, durchgeführt wird.

**Claims**

1. A process for obtaining a conjugated diolefin from a mixture of $C_4$ or $C_5$ hydrocarbons which contains this diolefin, by single-stage or multistage extractive distillation using a selective solvent and recycling the latter to the extractive distillation, after it has been obtained from the stripping zone downstream of each extractive distillation, a bleed stream of the recycled selective solvent being regenerated in a solvent purification stage in which the high-boiling or non-volatile impurities present in the solvent are separated off, and the regenerated solvent obtained from the solvent purification stage also being recycled, wherein the solvent to be regenerated is regenerated in a thin-film evaporation zone, using a stripping agent.

2. A process as claimed in Claim 1, wherein the selective solvent to be regenerated is fed into the upper third of a vertical thin-film evaporation zone, from which a top product comprising the regenerated solvent, and a bottom product containing the high-boiling or non-volatile impurities are taken off.

3. A process as claimed in Claims 1 and 2, wherein the stripping agent is fed into the lower third of the thin-film evaporation zone.

4. A process as claimed in Claims 1 to 3, wherein the stripping agent is fed into the bottom of the thin-film evaporation zone.

5. A process as claimed in Claims 1 to 4, wherein water or an organic solvent having a boiling point of from 30 to 200°C is used as the stripping agent.

6. A process as claimed in Claims 1 to 5, wherein part of the bottom product taken off from the thin-film evaporation zone is recycled to the bottom of this zone.

7. A process as claimed in Claims 1 to 6, wherein the regeneration is carried out in a vertical thin-film evaporator, using mechanically operated wipers which brush the inner wall of the thin-film evaporator.

## Revendications

1. Procédé de séparation d'une dioléfine conjuguée d'un mélange d'hydrocarbures en $C_4$ ou $C_5$ qui en contient par une extraction par distillation en un stade ou en plusieurs stades à l'aide d'un solvant sélectif avec recyclage du solvant sélectif, séparé dans une zone ou des zones d'entraînement disposée(s) en aval de chaque zone d'extraction par distillation, dans un stade d'extraction par distillation, un courant partiel du solvant sélectif à recycler étant régénéré dans un stade de purification par séparation des impuretés à point d'ébullition élevé ou non volatiles avant d'être recyclé, caractérisé en ce que le solvant à régénérer est traité dans une zone d'évaporation en couche mince en présence d'un agent d'entraînement.

2. Procédé suivant la revendication 1, caractérisé en ce que le solvant sélectif à régénérer est introduit dans le tiers supérieur d'une zone d'évaporation en couche mince verticale, de laquelle est soutiré comme produit de tête le solvant régénéré et comme résidu de l'évaporation un produit contenant les impuretés à point d'ébullition élevé ou non volatiles.

3. Procédé suivant l'une des revendications 1 ou 2, caractérisé en ce que l'agent d'entraînement est introduit dans le tiers inférieur de la zone d'évaporation en couche mince.

4. Procédé suivant l'une des revendications 1 à 3, caractérisé en ce que l'agent d'entraînement est introduit dans le résidu d'évaporation à la base de la zone d'évaporation en couche mince.

5. Procédé suivant l'une des revendications 1 à 4, caractérisé en ce que l'on emploie comme agent d'entraînement de l'eau ou un solvant organique avec un point d'ébullition dans la gamme de 30 à 200°C.

6. Procédé suivant l'une des revendications 1 à 5, caractérisé en ce qu'une fraction du résidu d'évaporation soutiré à la base de la zone d'évaporation en couche mince est ramenée en circuit fermé dans celle-ci.

7. Procédé suivant l'une des revendications 1 à 6, caractérisé en ce que la régénération est réalisée dans un évaporateur en couche mince vertical, équipé de racloirs actionnés par voie mécanique et raclant la paroi intérieure de l'évaporateur.